# EUROPÄISCHE PATENTSCHRIFT

(11) **EP 1 370 345 B1**
(45) Veröffentlichungstag und Bekanntmachung des Hinweises auf die Patenterteilung: **27.09.2006**
(21) Anmeldenummer: 02716841.8
(22) Anmeldetag: 22.03.2002
(51) Int. Cl.: B01D 61/18, B01D 63/02, C02F 1/00, C02F 9/00, A61L 2/02, B01D 35/04

(54) **FILTER ZUR GEWINNUNG VON IM WESENTLICHEN KEIMFREIEN WASSER**
FILTER USED IN THE PROVISION OF SUBSTANTIALLY GERM-FREE WATER
FILTRE D'ALIMENTATION D'EAU ESSENTIELLEMENT EXEMPTE DE GERMES

(30) Priorität: 23.03.2001 DE 10115633
(43) Veröffentlichungstag der Anmeldung: 17.12.2003
(73) Patentinhaber: FuMA-Tech Gesellschaft für funktionelle Membranen- und Anlagentechnologie mbH, 66386 St. Ingbert/Saar (DE)
(72) Erfinder: BAUER, Bernd, 71665 Vaihingen/Enz (DE); JOHANN, Jürgen, A-5310 Mondsee (AT); BINGGL, Gerd, A-5310 Mondsee (AT)
(74) Vertreter: Patentanwälte Ruff, Wilhelm, Beier, Dauster & Partner
(86) Internationale Anmeldenummer: PCT/EP2002/003220
(87) Internationale Veröffentlichungsnummer: WO 2002/076589

(56) Entgegenhaltungen:
- EP-A- 0 617 951
- EP-A- 0 709 341
- WO-A-93/02781
- GB-A- 2 329 633
- US-A- 4 636 307
- US-A- 5 045 198
- US-A- 5 705 067
- US-B1- 6 171 496
- PATENT ABSTRACTS OF JAPAN vol. 017, no. 533 (C-1114), 27. September 1993 (1993-09-27) & JP 05 146778 A (MITSUBISHI RAYON CO LTD), 15. Juni 1993 (1993-06-15)

## Beschreibung

Die vorliegende Erfindung betriff eine Vorrichtung zur Gewinnung von im wesentlichen keimfreiem Wasser, insbesondere vom legionellenfreiem Wasser, mit einem durchströmbaren Filtergehäuse mit einer Unfiltratseite an einem Wassereinlass und einer Filtratseite an einem Wasserauslass innerhalb dessen als Filter eine Vielzahl von porösen Hohlfasern angeordnet ist, die im Inneren der Vorrichtung mit mindestens einem Biozid versehen sind, und wobei die Hohlfaserwandungen aus einem Kunststoff bestehen, in den das Biozid eingebracht ist. Weiterhin betrifft die Erfindung ein Verfahren zur Gewinnung von legionellenfreiem Wasser.

Bei Legionellen handelt es sich um Umweltkeime, die nahezu in jedem Süßwasser-Bereich anzutreffen sind. Hier vermehren sie sich intrazellulär in Amöben. Unter bestimmten Voraussetzungen können sie jedoch beim Menschen Krankheiten hervorrufen. Hierbei handelt es sich insbesondere um die Legionärskrankheit, die sich entweder als Lungenentzündung oder als eine grippe-ähnliche Infektion äußert. Eine durch Legionellen verursachte Lungenentzündung verläuft häufig sehr schwer mit nicht selten tödlichem Ausgang. Darüber hinaus gehen viele Bronchialinfekte unerkanntermaßen auf eine Legionelleninfektion zurück.

Eine Infektion des Menschen verläuft in der Regel durch Einatmen von bakterienhaltigen kleinen Tröpfchen bzw. von Aerosolen. Solche Aerosole entstehen vornehmlich beim Duschen, wobei die Bakterien durch Einatmen der entstehenden Tröpfchen in die Lungen gelangen. In der Lunge vermehren sich die Legionellen intrazellulär in Monozyten und sind hier vor dem Zugriff der Immunabwehr des Menschen geschützt.

Die Legionellen finden optimale Lebensbedingungen in warmem Wasser. Sie bevorzugen hierbei besonders den Bereich zwischen 40 und 60°C. Es läßt sich jedoch in zunehmendem Maße beobachten, daß die Bakterien selbst bei Wassertemperaturen von bis zu 70°C noch überlebens- und vermehrungsfähig sind. Es kann also allgemein eine erhöhte Temperaturresistenz dieser gefährlichen Erreger festgestellt werden.

Da in Wasserleitungssystemen wie sie üblicherweise in Haushalten, Krankenhäusern, Altersheimen, Sportstätten etc. eingesetzt sind, Wassertemperaturen und Lebensbedingungen herrschen, die den Legionellen ein Überleben und Vermehren gestatten, ist es notwendig, Maßnahmen zu treffen, die gewährleisten, daß das beispielsweise zum Duschen verwendete Wasser von Legionellen frei ist. Herkömmlicherweise werden hierbei die Bakterien durch Überhitzung abgetötet. Da die Bakterien bisher einen Temperaturtoleranzbereich von etwa 40 bis etwa 60°C aufwiesen, war es zum Abtöten der Bakterien ausreichend, das Wasser auf etwa 80°C zu erhitzen. Da jedoch in letzter Zeit eine erhöhte Temperaturresistenz der Legionellen zu beobachten ist, ist eine solche Temperatur zum sicheren Abtöten der Bakterien nicht mehr ausreichend. Vielmehr wären nun Temperaturen von etwa 100°C notwendig, um zu gewährleisten, daß die Bakterien vollständig abgetötet werden. Ein Erhitzen des Wassers bis zum Siedepunkt ist jedoch in Wasserleitungssystemen, wie sie üblicherweise in Haushalten, Hotels, Sportstätten und ähnlichem verwendet werden, nicht möglich. Daher ist es notwendig geworden, die Legionellen auf andere Weise aus dem Wassersystem zu entfernen.

Die EP 617951 beschreibt eine tragbare Vorrichtung zur Reinigung von Flüssigkeiten, welche eine Sektion mit Aktivkohle und eine Sektion mit mikroporösen Hohlfasern umfasst. Die WO 93/02781 befasst sich mit einem tragbaren Notsystem zur Wassersterilisation, wobei die Filtration mit Hilfe von Hohlfasern erfolgt. Eine in der US 6,171,496 beschriebene Filtereinheit für die Mikrofiltration mit semipermeablen Hohlfasern ist für eine Ultrafiltration geeignet. Ein biologisches Fouling der Membran, insbesondere durch Bakterien, wird durch die Behandlung der Hohlfasern mit antimikrobiellen Wirkstoffen bzw. Einlagerungen solcher Wirkstoffe weitestgehend verhindert. Durch Ablagerungen auf den Hohlfasern haben die bekannten Filter eine relativ kurze Standzeit.

Die Erfindung stellt sich die Aufgabe, eine Vorrichtung und ein Verfahren bereitzustellen, mit welchem legionellenfreies Wasser über einen größeren Zeitraum gewonnen werden kann. An einer derartigen Vorrichtung bzw. an einem entsprechenden Verfahren besteht ein großes Bedürfnis, da diese gefährlichen Krankheitserreger durch deren zunehmende Temperaturresistenz mit herkömmlichen Methoden nicht mehr sicher abgetötet werden können. Die Aufgabe wird durch eine Vorrichtung gelöst, wie sie in Anpruch 1 beschrieben ist. Bevorzugte Ausführungsformen dieser Vorrichtung sind in den Ansprüchen 2 bis 30 beschrieben. Anspruch 31 beschäftigt sich mit einem Verfahren zur Gewinnung von legionellenfreiem Wasser. Der Wortlaut sämtlicher Ansprüche wird hiermit durch Bezugnahme zum Inhalt der Beschreibung gemacht.

Die erfindungsgemäße Vorrichtung, die zur Gewinnung von im wesentlichen keimfreiem Wasser, insbesondere von legionellenfreiem Wasser dient, weist zunächst ein durchströmbares Filtergehäuse auf. Dieses Filtergehäuse besitzt einen Wassereinlaß an der Unfiltratseite und einen Wasserauslaß an der Filtratseite. Innerhalb dieses Filtergehäuses ist eine Vielzahl von porösen Hohlfasern angeordnet. Wenn das Wasser diese Filtervorrichtung durchströmt, wird es durch die Wandung der Hohlfasern hindurchgeleitet. Aufgrund der entsprechenden Porosität der Hohlfasern werden hierbei Verunreinigungen des Wassers, insbesondere Bakterien, zurückgehalten, so daß das Wasser nach dem Durchströmen der Hohlfaserwandung im wesentlichen keimfrei und insbesondere legionellenfrei ist.

Die bevorzugten Anwendungsgebiete der erfindungsgemäßen Vorrichtung liegen vor allem in Hotels, Sportstätten, Krankenhäusern, Altersheimen und Einfamilienhäusern. Wassersysteme in derartigen Bereichen sind besonders von einem Legionellenbefall bedroht, da sich Legionellen besonders leicht in stehendem, warmem Wasser entwickeln können. Da in den Wassersystemen der genannten Bereiche häufig derartig stehendes Wasser auftreten kann, kann es hier zu massiven Legionellenvermehrungen kommen.

Wie bereits erwähnt, geschieht eine Infektion mit Legionellen vornehmlich durch das Einatmen von legionellenhaltigen Aerosolen. Solche Aerosole treten vornehmlich beim Duschen oder beim Gebrauch jedweder Brausen, zum Beispiel im Küchenbereich, auf. Weiterhin können diese gefährlichen Aerosole auch in Whirlpools auftreten. Die erfindungsgemäße Vorrichtung ist daher besonders für den Einsatz in Brausen und/oder Whirlpools geeignet.

Darüber hinaus ist die erfindungsgemäße Vorrichtung in zahlreichen weiteren Bereichen mit Vorteil einsetzbar, insbesondere in solchen Bereichen, wo eine allgemeine Erniedrigung der Keimzahl gefordert ist. Hierzu zählen viele pharmazeutische Anwendungen, die Aufbereitung von Dialysewasser und von Trinkwasser, beispielsweise unmittelbar am Wasserhahn oder unter der Spüle.

In einer bevorzugten Ausführungsform der erfindungsgemäßen Vorrichtung sind die Hohlfasern innerhalb des Filtergehäuses für das Anströmen von der äußeren Oberfläche her vorgesehen. Hierbei tritt das unfiltrierte Wasser, nachdem es durch den Wassereinlaß in das Filtergehäuse gelangt ist, mit der Wandung der Hohlfasern von außen in Kontakt und dringt über die entsprechenden Poren in das Innere der Hohlfasern vor. Dies hat den Vorteil, daß die Legionellen und andere Verunreinigungen des Wassers an der Außenseite der Hohlfasern verbleiben und nur das gefilterte Wasser durch das Innere der Hohlfasern läuft. Dadurch wird vermieden, daß das Innere der Hohlfasern verstopfen könnte.

Vorteilhafterweise ist mindestens eine Öffnung der Hohlfasern mit der Filtratseite verbunden, so daß das filtrierte Wasser innerhalb der Hohlfasern über den Wasserauslaß den Filter verlassen kann. Bevorzugterweise ist die Öffnung der Hohlfaser in einer Grundplatte angeordnet, die also den Wasserauslaß bilden kann. Vorzugsweise sind alle Öffnungen der verschiedenen Hohlfasern innerhalb des Filtergehäuses in einer gemeinsamen Grundplatte angeordnet. Hierbei können jeweils beide Öffnungen einer Hohlfaser oder lediglich eine Öffnung einer Hohlfaser in der Grundplatte angeordnet sein. Die Öffnungen der Hohlfaser können in die Grundplatte gesteckt und/oder mit ihr vergossen sein. Vorteilhafterweise können die Öffnungen auch mit der Grundplatte verklebt sein, beispielsweise mit Epoxidharz. In einer bevorzugten Ausführungsform übernimmt die Grundplatte zusätzlich eine Dichtungsfunktion. Hierfür kann die Grundplatte beispielsweise aus elastischem Material, insbesondere Kunststoff, bestehen.

In einer bevorzugten Ausführungsform der erfindungsgemäßen Vorrichtung weist das Filtergehäuse ein Gesamtaufnahmevolumen von etwa 25 bis etwa 1000 ml auf. Besonders bevorzugt ist ein Aufnahmevolumen von etwa 50 bis etwa 500 ml. Vorteilhafterweise ist die erfindungsgemäße Vorrichtung für einen Wasserdurchlaß (Permeatstrom) von etwa 100 bis etwa 4000 l/h, insbesondere etwa 500 bis etwa 3000 l/h ausgebildet. Dieser Wasserdurchlaß bezieht sich auf übliche Wasserdrücke von etwa 1 bis etwa 6 bar, wie sie normalerweise in Häusern vorhanden sind. Selbstverständlich reduziert sich der Wasserdurchlaß der erfindungsgemäßen Vorrichtung, wenn der im Wassersystem vorhandene Wasserdruck niedriger ist.

Bei der erfindungsgemäßen Vorrichtung ist der Wasserdurchlaß des Filters im ungebrauchten Zustand größer als der des Durchflußbegrenzers auf der Filtratseite des Gehäuses. Die erfindungsgemäße Vorrichtung ist daher zumindest im ungebrauchten Zustand überdimensioniert, so daß sie nicht ein limitierendes Element darstellt, welches den Wasserfluß behindern würde. Vorzugsweise ist der Wasserdurchlaß der erfindungsgemäßen Vorrichtung im ungebrauchten Zustand etwa 1,5 bis etwa 4 mal so groß wie der Durchlaß des Durchflußbegrenzers. Da im Verlauf des Gebrauchs der erfindungsgemäßen Vorrichtung die Verunreinigungen des Wassers, insbesondere die Legionellen, sich auf den Wandungen der Hohlfasern absetzen, wird sich der Wasserdurchlaß der erfindungsgemäßen Vorrichtung naturgemäß mit der Zeit verringern. Nach einer gewissen Gebrauchsdauer wird daher ein Zeitpunkt erreicht sein, zu dem der Wasserdurchlaß der erfindungsgemäßen Vorrichtung nicht mehr größer ist als der Wasserdurchlaß des Durchflußbegrenzers. Von da ab wird sich der Wasserdurchlaß des gesamten Systems langsam vermindern, so daß es unter Umständen notwendig sein kann, die erfindungsgemäße Vorrichtung zu erneuern oder auszutauschen.

Der Durchflußbegrenzer ist auf der Filtratseite des Filtergehäuses vorgesehen. Seine Durchgangsöffnung ist kleiner als die Durchgangsöffnung des noch ungebrauchten Filters. Da der Wasserdurchlaß des erfindungsgemäßen Filters mit der Zeit abnimmt, gewährleistet der Durchflußbegrenzer einen über einen langen Zeitraum gleichbleibenden Wasserstrom. Sollte der Wasserstrom dann jedoch langsam abnehmen, kann dies ein Hinweis darauf sein, daß der erfindungsgemäße Filter ausgetauscht und/oder erneuert werden sollte. Ein geeigneter Durchflußbegrenzer kann den Wasserstrom auf etwa ein Drittel begrenzen. Vorteilhafterweise kann der Durchflußbegrenzer auch variabel ausgestaltet sein, so daß sich die Begrenzung je nach Bedarf einstellen läßt. Hierfür kann eine einstellbare Blende vorgesehen sein, die sich beispielsweise an der dem Wasseraustritt entgegengesetzten Seite einer Handbrause befinden kann.

Ein weiterer Vorteil des Durchflußbegrenzers ist, daß durch den hierdurch reduzierten Permeatstrom die Standzeit der erfindungsgemäßen Vorrichtung erhöht wird. Beispielsweise kann es daher vorteilhaft sein, den Permeatstrom auf etwa 500 bis etwa 1500 l/min, insbesondere auf etwa 700 bis etwa 1100 l/min zu begrenzen.

Besonders vorteilhaft wirkt sich ein Durchflußbegrenzer auf der Filtratseite aus.

In einer besonders bevorzugten Ausführungsform der erfindungsgemäßen Vorrichtung weisen die Poren der Hohlfasern eine Porenweite von etwa 0,01 bis etwa 0,2 µm auf. Besonders vorteilhaft ist eine Porenweite von etwa 0,05 bis etwa 0,2 µm. Durch derartige Porenweiten wird sichergestellt, daß Legionellen zuverlässig aus dem Wasser herausfiltriert werden. Weiterhin werden hierdurch auch andere Verunreinigungen, wie verschiedene Schwebstoffe, Kalk oder ähnliches, aus dem Wasser entfernt. Darüber hinaus können andere Bakterien, wie beispielsweise Escherichia coli oder auch Viren, aus dem Wasser herausfiltriert werden.

Vorteilhafterweise weisen die Hohlfasern einen Innendurchmesser von etwa 10 bis etwa 200 µm, insbesondere etwa 50 bis etwa 150 µm, auf. Bei Hohlfasern mit derartigen Innendurchmessern in der erfindungsgemäßen Vorrichtung wird sichergestellt, daß zum einen ein ausreichender Wasserfluß innerhalb der Vorrichtung stattfinden kann und zum anderen eine ausreichende Anzahl von Hohlfasern innerhalb der Vorrichtung angeordnet sein kann.

In einer bevorzugten Ausführungsform der erfindungsgemäßen Vorrichtung weisen alle Hohlfasern innerhalb der Vorrichtung zusammen eine äußere Gesamtoberfläche von etwa 0,01 bis etwa 3 m², insbesondere etwa 0,05 bis etwa 1,5 m², auf. An dieser Oberfläche findet die Filtration des Wassers statt und daher ist die Oberfläche so bemessen, daß ein ausreichender Wasserdurchfluß gewährleistet ist. Andererseits wird durch die Oberfläche die Größe der erfindungsgemäßen Vorrichtung mitgeprägt. Daher ist die Oberfläche auch nicht zu groß, damit die Größe der gesamten Vorrichtung in einem praktikablen Rahmen bleibt. Besonders bevorzugt ist eine Oberfläche von etwa 0,09 bis etwa 1 m². Eine derartige Fläche stellt einen besonders guten Kompromiß zwischen beiden erwähnten Aspekten dar.

In einer besonders bevorzugten Ausführungsform der erfindungsgemäßen Vorrichtung bestehen die porösen Hohlfasern aus Kunststoff. Dieser Kunststoff kann ein Polymer oder ein Copolymer sein. Es kommen hierfür eine Vielzahl von herkömmlichen Kunststoffen in Frage. Voraussetzung für den Einsatz in der erfindungsgemäßen Vorrichtung ist, daß sich mit dem Kunststoff eine Hohlfaser herstellen läßt, die die entsprechenden Poren aufweist. Die Herstellung einer geeigneten Hohlfaser kann beispielsweise durch thermische Fällung eines aufgeschmolzenen Polymers mit einem Nichtlösemittel und Ausziehen des Polymers aus der Schmelze erfolgen. Das Innere der Hohlfaser wird mit Hilfe einer Spinndüse ausgebildet, die einen entsprechenden Luftstrom verursacht. Die Ausbildung der Poren innerhalb der Wandung der Hohlfasern erfolgt infolge der Eigenschaften des gewählten Kunststoffs und der Herstellungsbedingungen der Fasern. Besonders bevorzugte Substanzen für die Herstellung der Hohlfasern sind Polyvinylidenfluorid und/oder Polyethersulfon.

In einer besonders bevorzugten Ausführungsform der erfindungsgemäßen Vorrichtung bestehen die Hohlfasern zumindest teilweise aus einem lonenaustauschermaterial, insbesondere einer lonenaustauschermembran. Hierdurch werden weitere Substanzen aus dem Wasser entfernt, die die Wasserqualität beeinflussen können. Erfindungsgemäß besonders bevorzugt ist hierbei die Entfernung von Blei und/oder Nickel aus dem Wasser, wobei die Härte des Wassers vorzugsweise nicht weiter beeinflußt wird.

Weiterhin ist es bevorzugt, daß auf der Unfiltratseite ein Vorfilter angeordnet ist. Hierdurch können grobe Verunreinigungen, wie beispielsweise Rostpartikel oder Schwebstoffe, zurückgehalten werden, bevor sie mit den Hohlfasern in Kontakt treten. So können die Hohlfasern geschützt und die Lebensdauer der Vorrichtung verlängert werden. Als Vorfilter kann beispielsweise ein Kohlevlies oder, besonders bevorzugt, ein Mehrschichtvliesstoff eingesetzt sein. Insbesondere bei stark belastetem Wasser kann es vorteilhaft sein, daß der Vorfilter als Wegwerffilter vorgesehen ist, der bei Bedarf und unter Umständen unabhängig von der gesamten Vorrichtung getrennt ausgetauscht werden kann.

In einer bevorzugten Ausführungsform der erfindungsgemäßen Vorrichtung sind die Hohlfasern flexibel. Hierdurch können die Hohlfasern in unterschiedlicher Weise innerhalb der erfindungsgemäßen Vorrichtung angeordnet sein. Beispielsweise sind die Hohlfasern miteinander verflochten, verwoben oder verstrickt. In einer weiteren Ausführungsform sind die Hohlfasern zu einer Spule aufgedreht oder als ein Wickel ohne besondere Ordnung angeordnet.

Bevorzugterweise sind die Hohlfasern selbsttragend ausgebildet. Hierdurch können die Hohlfasern ohne weitere Maßnahmen dem Wasserdruck standhalten, ohne hierdurch in ihrer Anordnung wesentlich beeinflußt zu werden. Durch die selbsttragende Ausgestaltung werden weitere Befestigungen der Hohlfasern innerhalb der erfindungsgemäßen Vorrichtung im wesentlichen überflüssig.

In einer bevorzugten Ausführungsform der erfindungsgemäßen Vorrichtung ist die Grundplatte, innerhalb derer eine oder beide Öffnungen der Hohlfasern angeordnet sind, für den unmittelbaren Wasseraustritt ausgebildet. Dies kann beispielsweise die Wasseraustrittsöffnung einer Sanitärarmatur sein. Dies hat den Vorteil, daß zwischen der erfindungsgemäßen Vorrichtung und dem Wasseraustritt kein Raum entsteht, in welchem Wasser, insbesondere warmes Wasser, über einen längeren Zeitraum stehen könnte. Solches stehendes Wasser fördert die Entwicklung von Legionellen und anderen Erregern, weshalb solche Räume ein besonderes Risiko darstellen. Erfindungsgemäß ist es deshalb bevorzugt, daß die Filtervorrichtung dafür vorgesehen ist, möglichst nahe an einer Wasseraustrittsstelle angeordnet zu sein. Besonders vorteilhaft kann es jedoch sein, zusätzlich eine weitere erfindungsgemäße Vorrichtung in zentraler Position, beispielsweise im oder nahe des Heißwasserspeichers, anzubringen. Hierdurch können die Verunreinigungen aus dem Wasser entfernt werden, bevor sie in das periphere Wassersystem gelangen, wo sie eventuell zu einer Verunreinigung des weitverzweigten Leitungsnetzes führen könnten. Eine oder mehrere erfindungsgemäße Vorrichtungen an den Wasseraustrittsstellen oder nahe dieser Austrittsstellen gewährleisten, daß eventuell verschleppte Legionellen, die sich stromabwärts des ersten zentralen Filters sogar vermehrt haben könnten, sicher aus dem Wasser entfernt werden.

Vorteilhafterweise ist die erfindungsgemäße Vorrichtung als Einwegmaterial (Disposible), also zum Einmalgebrauch, vorgesehen. Durch das Absetzen von Verunreinigungen auf den Hohlfasern und durch ein eventuelles Verkalken der Filtervorrichtung hat die erfindungsgemäße Vorrichtung nur eine begrenzte Lebensdauer. Ein Reinigen und/oder Austauschen der Hohlfasern kann sehr umständlich und aufwendig sein, so daß es vorteilhaft ist, die komplette Vorrichtung auszutauschen. Wenn die Filterkapazität der Hohlfaseroberflächen erschöpft ist, wird daher die erfindungsgemäße Vorrichtung vorzugsweise vollständig aus dem Wassersystem entfernt und entsorgt. Hierbei ist bevorzugt, daß die erfindungsgemäße Vorrichtung möglichst wenig zusätzliche Teile wie beispielsweise Dichtungen oder ähnliches aufweist, um so verhältnismäßig wenig Abfall zu produzieren.

Die gebrauchte Filtervorrichtung stellt in gewisser Weise ein infektiöses Material dar, da Verunreinigungen des Wassers, insbesondere Bakterien, zunächst lediglich herausfiltriert und nicht abgetötet werden. Dies ist jedoch unproblematisch, da Bakterien oder ähnliches in der erfindungsgemäßen Filtervorrichtung im allgemeinen keine Bedingungen vorfinden, die ein Überleben oder Vermehren fördern. Zum anderen ist eine Infektion mit Legionellen beispielsweise ausgeschlossen, da beim Austausch der erfindungsgemäßen Filtervorrichtung keine Aerosole entstehen und somit keine Inhalation dieser Bakterien stattfinden kann. Zusätzlich sind beim Austausch der Filtervorrichtung die verschmutzten Hohlfasern komplett vom Filtergehäuse umgeben, so daß es zu keinem Kontakt mit den Verunreinigungen kommen kann. Um ein absolut gefahrloses Austauschen der Filtervorrichtung zu gewährleisten, kann es beispielsweise vorteilhaft sein, die Filtervorrichtung mit einer Desinfektionslösung zu behandeln.

In einer weiteren bevorzugten Ausführungsform ist die erfindungsgemäße Vorrichtung auf der Unfiltratseite durch ein Ventil, insbesondere ein Rückschlagventil, abgedichtet. Hierdurch wird eine weitere Schutzmaßnahme für das Auswechseln der gebrauchten Vorrichtung bereitgestellt. Zum anderen wird eine rückwärtige Verkeimung vermieden.

Bei der erfindungsgemäßen Vorrichtung ist das Innere der Vorrichtung mit mindestens einem Biozid versehen. Die sich auf dem Filter erfindungsgemäß absetzenden Keime, insbesondere Legionellen, können sich hier prinzipiell vermehren und Kolonien bilden. Hierdurch kann die Gefahr einer Rückwärtsinfektion des Wassersystems entstehen. Erfindungsgemäß ist es deshalb vorgesehen, die Filtervorrichtung derart mit Biozid zu versehen, daß die sich im Filter absetzenden Keime zuverlässig abgetötet werden. Die Hohlfasern werden im Inneren der Vorrichtung mit dem Biozid versehen. Als geeignete Biozide kommen alle für Trinkwasser zugelassenen Biozide in Frage. Besonders bevorzugt ist die Verwendung von Polyelektrolyten, die von den Hohlfasern zurückgehalten werden. Beispiele für geeignete Biozide sind Polyhexamethylenguanidiniumsalz, vorzugsweise als Phosphat, Chlorhexidin und/oder Vantosil^{R}.

Vorteilhafterweise ist das Biozid ein immobilisiertes Biozid mit Depotwirkung. Hierdurch kann sichergestellt werden, daß das Biozid über einen langen Zeitraum seine Wirkungsamkeit entfaltet und so alle Keime innerhalb der Filtervorrichtung zuverlässig abtötet.

Gemäß der Erfindung wird das oder die Biozide bereits bei der Herstellung der Hohlfasern auf oder in die Fasern eingebracht. Hierfür können die Biozide bereits während der Herstellung der Hohlfasern in das jeweilige Material, insbesondere in den jeweiligen Kunststoff, eingebracht werden und so in die Wandung der herzustellenden Hohlfasern eingebracht sein. Dies hat den Vorteil, daß nicht nur die äußere Oberfläche der Hohlfasern mit dem Biozid versehen ist, sondern daß ebenfalls die innere Oberfläche sowie auch die Poren mit Biozid ausgestattet sind. Hierdurch wird ein Durchwachsen von Keimen durch die Poren vollständig unterbunden.

In einer besonders vorteilhaften Ausführungsform der erfindungsgemäßen Vorrichtung erstrecken sich die Hohlfasern vom Wassereinlaß zum Wasserauslaß des Filtergehäuses und sind an der Unfiltratseite, also im Bereich des Wassereinlasses, geschlossen. Die Hohlfasern können beispielsweise durch eine Verklebung oder Verschweißung geschlossen sein. Hierbei sind die Hohlfasern im Filtergehäuse vorzugsweise im wesentlichen parallel angeordnet, so daß sie ein lang gestrecktes Bündel von Hohlfasern bilden. Hierbei kann es bevorzugt sein, daß die Hohlfasern ihrer Länge nach miteinander verklebt und/oder vergossen sind. Ein derartiges Bündel besteht beispielsweise aus etwa 10 bis etwa 100 Hohlfasern, insbesondere etwa 30 bis etwa 60 Hohlfasern. Diese Hohlfasern sind vorzugsweise in einem lang gestreckten Filtergehäuse angeordnet. In einer bevorzugten Ausführungsform weisen die Hohlfasern eine Länge von etwa 10 bis etwa 200 cm, insbesondere etwa 30 bis etwa 100 cm auf.

Bevorzugterweise ist das Filtergehäuse ein Brauseschlauch oder das Filtergehäuse ist für das Einbringen in einen Brauseschlauch ausgebildet. Vorzugsweise kann die erfindungsgemäße Vorrichtung im wesentlichen aus den Hohlfasern bestehen, die für das Einbringen in einen Brauseschlauch als Filtergehäuse vorgesehen sind. Hierbei kann die Länge der Hohlfasern mit der Länge des jeweiligen Brauseschlauchs zusammenhängen. Besonders bevorzugt ist es, wenn der ganze Brauseschlauch der Länge nach mit Hohlfasern entsprechender Länge versehen ist. Die erfindungsgemäße Vorrichtung in Form eines Brauseschlauchs bzw. innerhalb eines Brauseschlauchs hat den Vorteil, daß die Filterwirkung der Vorrichtung sehr nah an der Wasseraustragsstelle stattfindet. Hierdurch wird vermieden, daß stromabwärts von der Filtervorrichtung Räume mit stehendem Wasser auftreten können, die die Vermehrung von Legionellen fördern könnten. In dieser Ausführungsform befindet sich die erfindungsgemäße Vorrichtung vorteilhafterweise sehr nah am Gebrauchsort des Wassers, wodurch eine erneute Kontamination des Wassers nach der Filtrierung verhindert wird.

Es kann vorgesehen sein, daß der Brauseschlauch, der die Hohlfasern enthält, als Wegwerfschlauch konzipiert ist. Hierdurch wird das Austauschen der erfindungsgemäßen Vorrichtung bei nicht mehr ausreichendem Wasserdurchlaß sehr vereinfacht. Der Schlauch wird dann bei Bedarf abmontiert, insbesondere abgeschraubt, und so wie er ist entsorgt. Andererseits kann es bevorzugt sein, daß die Füllung des Schlauches, also insbesondere die Hohlfasern, bei Bedarf entfernt, insbesondere herausgezogen, werden und durch neue Hohlfasern ersetzt werden.

In einer besonders bevorzugten Ausführungsform reichen die Hohlfasern der erfindungsgemäßen Vorrichtung bis in den Brausekopf hinein. Hierdurch werden wiederum Räume vermieden, in denen warmes Wasser stehen könnte. In dieser Ausführungsform können die Hohlfasern direkt in der Düsenplatte, also der Grundplatte, enden, so daß das Wasser unmittelbar aus den Hohlfasern austritt. Auch diese Brause, vorzugsweise inklusive Schlauch, kann als Wegwerfbrause vorgesehen sein, um den Austausch der Filtervorrichtung zu erleichtern.

In einer weiteren besonders bevorzugten Ausführungsform sind beide Öffnungen der Hohlfasern mit der Filtratseite verbunden. Hierbei sind die Öffnungen vorzugsweise in einer gemeinsamen Grundplatte angeordnet. Vorzugsweise sind die Hohlfasern als Schlaufen angeordnet. In dieser Ausführungsform der erfindungsgemäßen Vorrichtung können etwa 250 bis etwa 1000 Hohlfasern, insbesondere etwa 500 bis etwa 800 Hohlfasern, im Filtergehäuse vorgesehen sein. Bevorzugterweise weisen die Hohlfasern eine Länge von etwa 5 bis etwa 50 cm, insbesondere etwa 10 bis etwa 25 cm auf. Die Länge der Hohlfasern bestimmt hier die Länge der erfindungsgemäßen Vorrichtung mit. Da in dieser Ausführungsform die Hohlfasern vorzugsweise als Schlaufen angeordnet sind, kann die Filtervorrichtung eine Länge von etwa der Hälfte der Länge der Hohlfasern aufweisen.

Besonders bevorzugt ist es, daß das Filtergehäuse zusammen mit den Hohlfasern eine auswechselbare Einheit bildet. Hierbei handelt es sich insbesondere um eine Kartusche. Eine solche Einheit kann an geeigneter Stelle im Wassersystem eingebracht werden und bei Bedarf ausgetauscht werden. Da hier die Hohlfasern, die eventuell mit infektiösen Verunreinigungen aus dem Wasser verschmutzt sind, vom Filtergehäuse umgeben sind, kommt der Anwender nicht mit den verunreinigten Hohlfasern in Berührung, so daß eine Infektionsgefahr beim Austauschen der erfindungsgemäßen Vorrichtung nicht besteht.

Eine solche Einheit kann für den Einbau in unterschiedlichen Stellen des Wasserleitungssystems vorgesehen sein. Besonders bevorzugt ist ein Einbau möglichst nahe am Benutzungsort des Wassers, wodurch eine erneute Verkeimung in weiterführende Leitungen ausgeschlossen ist. Es kann weiterhin sehr vorteilhaft sein, eine erfindungsgemäße Filtereinrichtung zentral anzubringen, und eine weitere Vorrichtung in unmittelbarer Nähe des Wasseraustritts zu installieren. Bei einem Mischventil kann die erfindungsgemäße Vorrichtung beispielsweise in der Heißwasserleitung und/oder in der Mischwasserleitung eingebaut sein.

Besonders vorteilhaft ist es, wenn die erfindungsgemäße Einheit für den Einbau in eine Brause, beispielsweise in eine Kopfbrause und/oder in eine Handbrause vorgesehen ist. Um eine möglichst platzsparende Ausbildung der erfindungsgemäßen Vorrichtung zu gewährleisten, kann es vorteilhaft sein, die Hohlfasern als einen relativ engen Wickel anzuordnen. Die erfindungsgemäße Vorrichtung kann auch in dieser Ausführungsform zum Austauschen vorgesehen sein oder die gesamte Brause ist als Wegwerfbrause ausgebildet.

Weiterhin umfaßt die Erfindung eine Vorrichtung zur Gewinnung von legionellenfreiem Wasser, die in ein Wassersystem eingebaut ist. Bezüglich der weiteren Merkmale der Vorrichtung wird auf die obige Beschreibung verwiesen. Vorzugsweise ist die erfindungsgemäße Vorrichtung zwischen einer Wassererwärmungsstelle und einer Wasseraustrittsstelle, beispielsweise einer Brause, eingebaut. In einer weiteren bevorzugten Ausführungsform der Erfindung ist die Wasseraustrittsstelle eine solche eines Whirlpools. Besonders bevorzugt ist es, daß die Filtervorrichtung möglichst nahe an einer Wasseraustrittsstelle angeordnet ist. Weiterhin ist es vorteilhaft, wenn mehrere Filtervorrichtungen im Wassersystem eingebaut sind, wobei zumindest eine der Filtervorrichtungen vorzugsweise möglichst nahe an einer Wasseraustrittsstelle angeordnet ist.

Schließlich umfaßt die Erfindung ein Verfahren zur Gewinnung von im wesentlichen keimfreiem Wasser, insbesondere von legionellenfreiem Wasser, wobei Wasser, insbesondere Warmwasser, filtriert, insbesondere ultrafiltriert, wird. Mit diesem Verfahren kann mikrobiologisch entlastetes Wasser gewonnen werden. Das Verfahren dient beispielsweise der Reduktion von Keimen in Trink-, Produkt- oder Prozeßwasser. Diese Filtration wird mit Hilfe mindestens einer Vorrichtung durchgeführt, wie sie oben beschrieben ist. Bezüglich der verschiedenen Merkmale dieser Vorrichtung wird auf das oben gesagte Bezug genommen.

Einzelheiten zu den beschriebenen Merkmalen und zu weiteren Merkmalen der Erfindung ergeben sich aus der nachfolgenden Beschreibung der Figuren in Verbindung mit den Beispielen und den Unteransprüchen. Hierbei können die einzelnen Merkmale jeweils für sich oder in Kombination miteinander verwirklicht sein.

In den Figuren ist gezeigt:

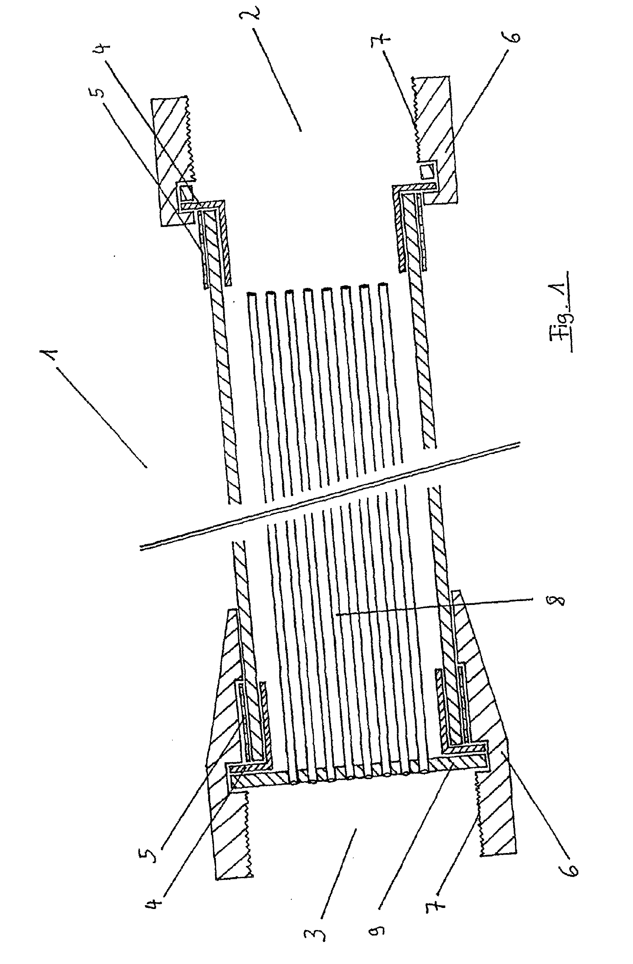

- Figur 1:: Ein erfindungsgemäßer Brauseschlauch im Längsschnitt,

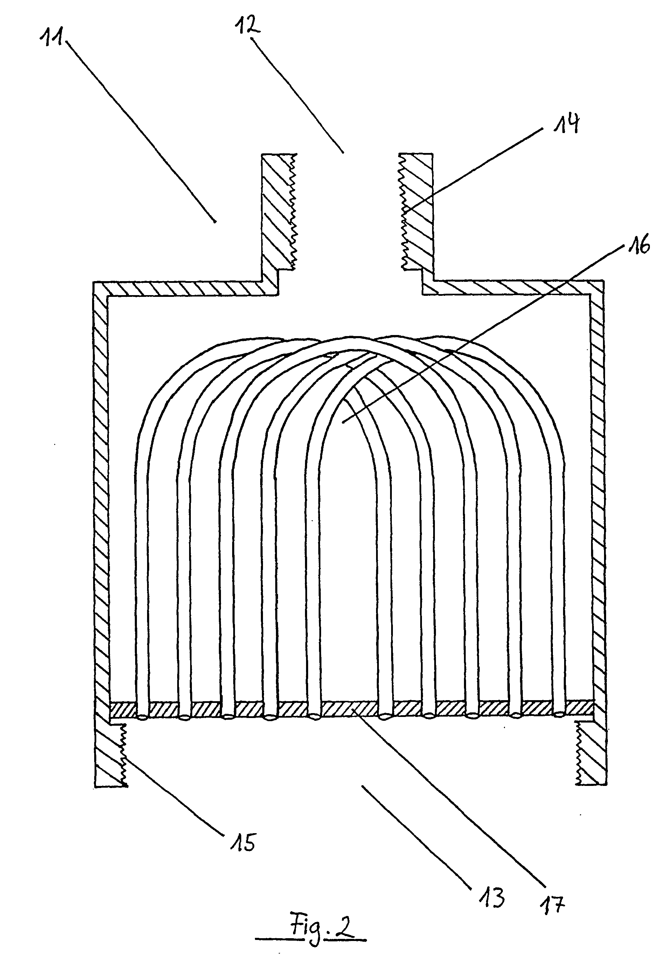

- Figur 2:: Eine erfindungsgemäße Kartusche im Längsschnitt,
- Figur 3:: Elektroneumikroskopische Querschnitte einer Hohlfaser,
- Figur 4:: Außenansicht und Längsschnitt durch ein Ausführungsbeispiel einer erfindungsgemäßen Brause.

Der erfindungsgemäße Brauseschlauch 1 weist auf der einen Seite einen Wassereinlaß 2 und auf der anderen Seite einen Wasserauslaß 3 auf. An beiden Öffnungsseiten des Schlauches ist jeweils ein umlaufender Nippel 4 sowie eine Preßhülse 5 angeordnet. Eine Überwurfmutter 6, die konisch ausgebildet sein kann, mit einem Gewinde 7 stellt zu beiden Seiten des Schlauches die Verbindung zu anschließenden Teilen (nicht dargestellt) her. Eine Vielzahl von Hohlfasern 8 ist innerhalb des Brauseschlauchs 1 angeordnet. Das dem Wassereinlaß 2 zugewandte Ende der Hohlfasern 8 ist geschlossen. Das dem Wasserauslaß 3 zugewandte Ende der Hohlfasern 8 ist offen. Diese Öffnungen der Hohlfasern 8 sind in einer Grundplatte 9 angeordnet, welche gleichzeitig eine Dichtung ist.

Eine Kartusche 11 weist einen Wassereinlaß 12 und einen Wasserauslaß 13 auf. Der Wassereinlaß 12 und der Wasserauslaß 13 sind jeweils mit einem Gewinde 14 und 15 versehen. Innerhalb der Kartusche 11 befindet sich eine Vielzahl von Hohlfasern 16, die als Schlaufen angeordnet sind. Die Öffnungen der Hohlfasern 16 sind in einer Grundplatte 17 angeordnet.

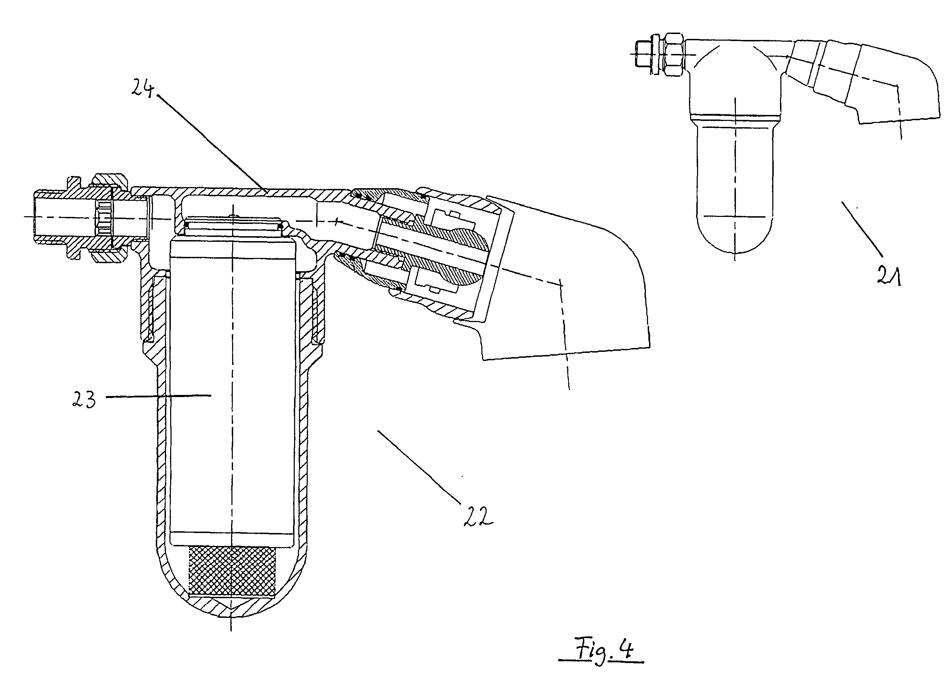

Figur 4 zeigt eine Ausführungsform einer erfindungsgemäßen Brause als Außenansicht 21 und als Längsschnitt 22. Die Filtervorrichtung 23 ist senkrecht zum Brausenkörper 24 angeordnet.

### Ausführungsbeispiele

### Beispiel 1

Der erfindungsgemäße Brauseschlauch 1 wird mit der Seite des Wassereinlasses 2 mit Hilfe der Überwurfmutter 6 mit dem Wasserleitungssystem verbunden. An der Seite des Wasserauslasses 3 wird eine geeignete Armatur, beispielsweise eine Handbrause, befestigt. Bei laufendem Wasser gelangt das Wasser zunächst mit den verschlossenen Öffnungen der Hohlfasern 8 in Kontakt. Über die äußeren Oberflächen der Hohlfasern 8 dringt das Wasser durch die Poren der Hohlfasern in das Innere der Hohlfasern und gelangt so in Richtung der Grundplatte 9. Die Grundplatte 9 weist eine Dicke von etwa 3 bis 4 mm auf. Sie besteht aus elastischem Kunststoff und dichtet dieses Ende der Vorrichtung ab. An der Grundplatte 9 tritt das Wasser aus den Hohlfasern 8 und damit auch aus dem Brauseschlauch 1 aus und gelangt in die hier angefügte Armatur, beispielsweise die Handbrause. Hier tritt das gefilterte Wasser aus dem Wasserleitungssystem aus.

### Beispiel 2

Die Kartusche 11 wird mit Hilfe der Gewinde 14 und 15 in ein Wasserleitungssystem eingefügt, wobei die Wasserstromrichtung vom Wassereinlaß 12 bis zum Wasserauslaß 13 verläuft. Nachdem das Wasser über den Wassereinlaß 12 in die Kartusche 11 gelangt, tritt es mit den äußeren Oberflächen der Hohlfasern 16 zunächst im hinteren Bereich der Schlaufen in Kontakt. Hier dringt es über die Poren der Hohlfasern 16 in das Innere der Hohlfasern ein, wobei die Verunreinigungen des Wassers, insbesondere die Legionellen, auf der Außenseite der Hohlfasern verbleiben. Über das Innere der Hohlfasern gelangt das filtrierte Wasser in Richtung der Grundplatte 17. Die Grundplatte 17 weist eine Dicke von etwa 3 bis 4 mm auf. Hier tritt das Wasser aus den Hohlfasern 16 aus und verläßt die Kartusche 11. Im Verlauf der Gebrauchsdauer der erfindungsgemäßen Vorrichtungen setzen sich verschiedene Verunreinigungen auf den Hohlfasern und innerhalb der Filtervorrichtungen ab. Dies führt mit der Zeit zu einer Beeinträchtigung der Filtrationsleistung. Wenn dies den Wasserdurchlaß zu stark beeinträchtigt, etwa nach 1 bis 1 1/2 Jahren Gebrauchsdauer, wird die komplette Filtervorrichtung ausgetauscht.

### Beispiel 3

Zur Untersuchung der Keimrückhaltung der erfindungsgemäßen Vorrichtung wird die Filtervorrichtung mit E.coli-haltigen Lösungen bespült.

E.coli K12 wird in Standard-I-Nährboullon bei 37 °C über 5 Stunden angezogen. Die Kultur wird auf 5x10⁷ Zellen/ml in 0,9% NaCl verdünnt. 150 ml dieser Bakteriensuspension wird in die Filtervorrichtung eingespült und verschiedene Proben genommen. Die Proben werden jeweils auf der Unfiltratseite (vor) und auf der Filtratseite (nach) zu verschiedenen Zeitpunkten entnommen, d.h. zum Zeitpunkt 0 Minuten, 2 Minuten und 5 Minuten. Die entnommenen Proben werden in BRILA-Bouillon angereichert bei einer Inkubation unter Schütteln bei 36 °C. Nach 24 bis 48 Stunden wird das Wachstum der Kulturen beurteilt. Weiterhin werden Anreicherungskulturen auf Selektiv-Nährböden ausgestrichen und bei 36 °C für 24 bis 48 Stunden bebrütet. Der eingesetzte E.coli K12 zeigte hierbei jeweils dunkelrote, glänzende Kolonien (Koloniemorphologie) auf dem Selektiv-Nährboden in einer weiteren Kontrolle. Die folgende Tabelle faßt die Ergebnisse dieses Filterdesintegritätstestes zusammen.

**Tabelle 1: Ergebnisse und Beurteilung des Wachstums von E. coli K12 in Anreicherungs- und Plattenkulturen von den Proben des Filterdesintegritätstests**

| Probenbezeichnung | Zeit (Tage) | Wachstum nach 24h in Anreicherungskultur | Wachstum nach 48h Anreicherungskultur | Koloniemorphologie auf Selektiv-Nährboden |
|---|---|---|---|---|
| Null-vor | 1 | - | - | - |
| Null-nach | 1 | - | - | - |
| 2 min-vor | 1 | + | + | +, E. coli |
| 2 min-nach | 1 | - | - | - |
| 5 min-vor | 1 | + | + | +, E. coli |
| 5 min-nach | 1 | - | - | - |
| | | | | |
| 0 min-vor | 3 | + | + | +, E. coli |
| 0 min-nach | 3 | - | + | -, kein E. coli *) |
| 2 min-nach | 3 | - | - | - |
| | | | | |
| 0 min-vor | 8 | - | + | -, kein E. coli *) |
| 0 min-nach | 8 | - | + | -, kein E. coli *) |
| 2 min-nach | 8 | - | - | - |
| | | | | |
| 0 min-vor | 15 | + | + | +, E. coli |
| 0 min-nach | 15 | - | - | - |
| 2 min-nach | 15 | - | - | - |
| | | | | |
| Rückspülung 500 ml | 18 | + | + | -, kein E. coli |

| | | | | |
|---|---|---|---|---|
| +: Wachstum, -: kein Wachstum, ±: nicht eindeutig zu beurteilendes Wachstum *) angereicherte Mikroorganismen der Anreicherungskulturen wuchsen nicht auf dem verwendeten Selektiv-Nährboden an. In einem parallelen Ausstrich auf Vollmedium wuchsen diese Mikroorganismen. Es handelt sich damit nicht um den eingesetzten E. coli K12, der auf den Filter aufgebracht wurde, sondern wahrscheinlich um einen Wasserkeim aus unserer Leitung | | | | |

### Beispiel 4

Die nachfolgende Tabelle gibt die verschiedenen Eigenschaften eines Ausführungsbeispiels einer Hohlfaser wieder.

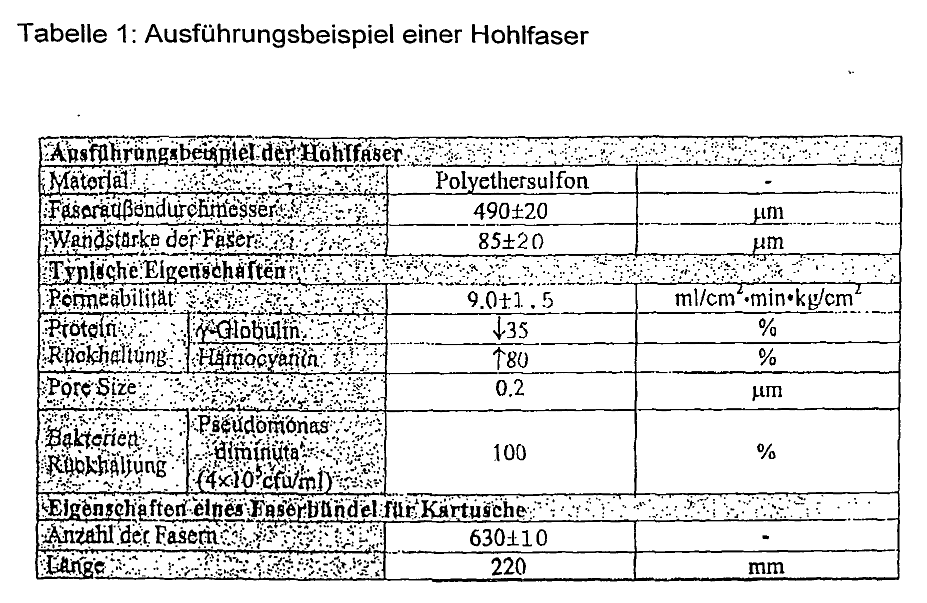

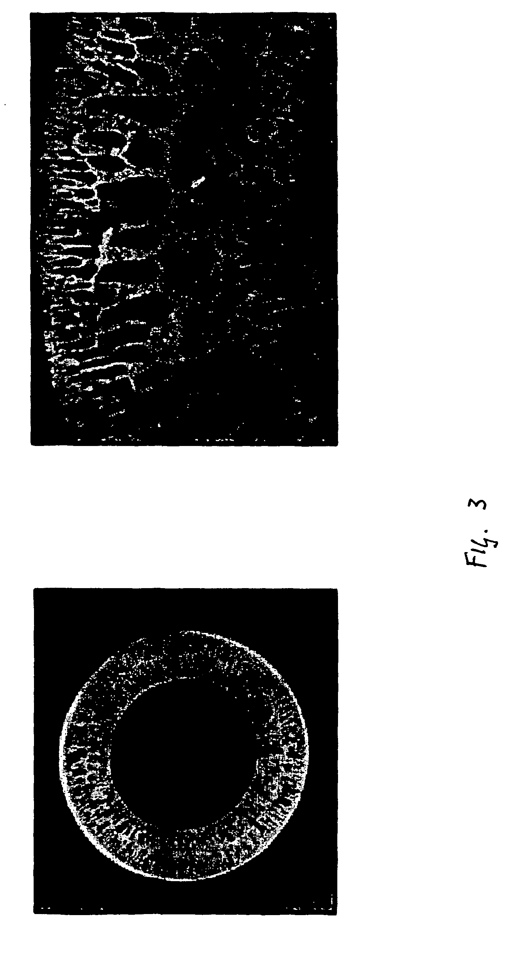

Die Figur 3 zeigt einen elektronenmikroskopischen Querschnitt durch eine Hohlfaser in zwei unterschiedlichen Vergrößerungen. Hierbei wird die Porosität der Wandung der Hohlfaser deutlich.

## Patentansprüche

1. Vorrichtung (1, 11) zur Gewinnung von im wesentlichen keimfreiem Wasser, insbesondere vom legionellenfreiem Wasser, mit einem durchströmbaren Filtergehäuse mit einer Unfiltratseite an einem Wassereinlaß (2, 12) und einer Filtratseite an einem Wasserauslaß (3, 13), innerhalb dessen als Filter eine Vielzahl von porösen Hohlfasern (8, 16) angeordnet ist, die im Inneren der Vorrichtung mit mindestens einem Biozid versehen sind, **dadurch gekennzeichnet, dass** auf der Filtratseite des Gehäuses ein Durchflußbegrenzer vorgesehen ist, dessen Durchgangsöffnung kleiner ist als die Durchgangsöffnung des noch ungebrauchten Filters.

2. Vorrichtung nach Anspruch 1, **dadurch gekennzeichnet, dass** das Biozid ein immobilisiertes Biozid mit Depotwirkung ist.

3. Vorrichtung nach Anspruch 1 oder Anspruch 2, **dadurch gekennzeichnet, dass** die Hohlfasern (8, 16) für das Anströmen von der äußeren Oberfläche her vorgesehen sind.

4. Vorrichtung nach einem der vorhergehenden Ansprüche, **dadurch gekennzeichnet, dass** mindestens eine Öffnung der Hohlfasern mit der Filtratseite verbunden ist, wobei vorzugsweise die Öffnung in einer vorzugsweise gemeinsamen Grundplatte (9, 17) angeordnet ist.

5. Vorrichtung nach einem der vorhergehenden Ansprüche, **dadurch gekennzeichnet, dass** das Filtergehäuse ein Gesamtaufnahmevolumen von etwa 25 bis etwa 1000 ml, insbesondere etwa 50 bis etwa 500 ml, aufweist.

6. Vorrichtung nach einem der vorhergehenden Ansprüche, **dadurch gekennzeichnet, dass** sie für einen Wasserdurchlaß von etwa 100 bis etwa 4000 l/h, insbesondere etwa 500 bis etwa 3000 l/h, ausgebildet ist.

7. Vorrichtung nach einem der vorhergehenden Ansprüche, **dadurch gekennzeichnet, dass** ihr Wasserdurchlaß im ungebrauchten Zustand größer ist, vorzugsweise 1,5 bis 4 mal so groß ist, als eine den Durchfluß begrenzende Stelle im Wassersystem.

8. Vorrichtung nach einem der vorhergehenden Ansprüche, **dadurch gekennzeichnet, dass** die Poren der Hohlfasern eine Porenweite von etwa 0,01 bis etwa 0,2 µm, insbesondere etwa 0,05 bis etwa 0,2 µm, aufweisen.

9. Vorrichtung nach einem der vorhergehenden Ansprüche, **dadurch gekennzeichnet, dass** die Hohlfasern einen Innendurchmesser von etwa 10 bis etwa 200 µm, insbesondere etwa 50 bis etwa 150 µm, aufweisen.

10. Vorrichtung nach einem der vorhergehenden Ansprüche, **dadurch gekennzeichnet, dass** die Hohlfasern einer Vorrichtung eine äußere Gesamtoberfläche von etwa 0,01 bis etwa 3 m², insbesondere etwa 0,05 bis etwa 1,5 m², aufweisen.

11. Vorrichtung nach einem der vorhergehenden Ansprüche, **dadurch gekennzeichnet, dass** die porösen Hohlfasern aus Kunststoff, insbesondere aus Polyvinylidenfluorid und/oder Polyethersulfon, bestehen.

12. Vorrichtung nach einem der vorhergehenden Ansprüche, **dadurch gekennzeichnet, dass** die Vorrichtung auf der Unfiltratseite einen Vorfilter aufweist.

13. Vorrichtung nach einem der vorhergehenden Ansprüche, **dadurch gekennzeichnet, dass** die Hohlfasern flexibel sind.

14. Vorrichtung nach einem der vorhergehenden Ansprüche, **dadurch gekennzeichnet, dass** die Hohlfasern selbsttragend ausgebildet sind.

15. Vorrichtung nach einem der vorhergehenden Ansprüche, **dadurch gekennzeichnet, dass** die Hohlfaserwandungen aus einem Kunststoff bestehen, in den das Biozid eingebracht ist.

16. Vorrichtung nach einem der vorhergehenden Ansprüche, **dadurch gekennzeichnet, dass** sich die Hohlfasern vom Wassereinlass (2) zum Wasserauslass (3) erstrecken und an der Unfiltratseite geschlossen sind.

17. Vorrichtung nach einem der vorhergehenden Ansprüche, insbesondere nach Anspruch 16, **dadurch gekennzeichnet, dass** die Hohlfasern (8) im Filtergehäuse im wesentlichen parallel angeordnet sind.

18. Vorrichtung nach einem der vorhergehenden Ansprüche, insbesondere nach Anspruch 16 oder Anspruch 17, **dadurch gekennzeichnet, dass** etwa 10 bis etwa 100 Hohlfasern, insbesondere etwa 30 bis etwa 60 Hohlfasern, vorgesehen sind, die insbesondere in einem langgestreckten Filtergehäuse angeordnet sind.

19. Vorrichtung nach einem der vorhergehenden Ansprüche, insbesondere nach einem der Ansprüche 16 bis 18, **dadurch gekennzeichnet, dass** die Hohlfasern eine Länge von etwa 10 bis etwa 200 cm, insbesondere etwa 30 bis etwa 100 cm, aufweisen.

20. Vorrichtung nach einem der vorhergehenden Ansprüche, insbesondere nach einem der Ansprüche 16 bis 19, **dadurch gekennzeichnet, dass** das Filtergehäuse ein Brauseschlauch (1) ist oder für das Einbringen in einen Brauseschlauch ausgebildet ist.

21. Vorrichtung nach einem der vorhergehenden Ansprüche, insbesondere nach einem der Ansprüche 1 bis 15, **dadurch gekennzeichnet, dass** beide Öffnungen der Hohlfasern (16) mit der Filtratseite verbunden sind.

22. Vorrichtung nach einem der vorhergehenden Ansprüche, insbesondere nach Anspruch 21, **dadurch gekennzeichnet, dass** die Hohlfasern (16) als Schlaufen angeordnet sind.

23. Vorrichtung nach einem der vorhergehenden Ansprüche, insbesondere nach Anspruch 21 oder Anspruch 22, **dadurch gekennzeichnet, dass** etwa 250 bis etwa 1000 Hohlfasern, insbesondere etwa 500 bis etwa 800 Hohlfasern, im Filtergehäuse vorgesehen sind.

24. Vorrichtung nach einem der vorhergehenden Ansprüche, inbesondere nach einem der Ansprüche 21 bis 23, **dadurch gekennzeichnet, dass** die Hohlfasern eine Länge von etwa 5 bis etwa 50 cm, insbesondere etwa 10 bis etwa 25 cm, aufweisen.

25. Vorrichtung nach einem der vorhergehenden Ansprüche, inbesondere nach einem der Ansprüche 21 bis 24, **dadurch gekennzeichnet, dass** das Filtergehäuse als eine mit den Hohlfasern auswechselbare Einheit, insbesondere als Kartusche (11), ausgebildet ist.

26. Vorrichtung nach einem der vorhergehenden Ansprüche, insbesondere nach einem der Ansprüche 21 bis 25, **dadurch gekennzeichnet, dass** sie für den Einbau in eine Brause vorgesehen ist.

27. Vorrichtung nach einem der vorhergehenden Ansprüche, **dadurch gekennzeichnet, dass** sie zum Einbau in ein Wassersystem, insbesondere in ein Warmwassersystem, ausgebildet ist, vorzugsweise zwischen einer Wassererwärmungsstelle und einer Wasseraustrittsstelle.

28. Vorrichtung nach Anspruch 27, **dadurch gekennzeichnet, dass** die Wasseraustrittsstelle eine Brause ist.

29. Vorrichtung nach Anspruch 27 oder Anspruch 28, **dadurch gekennzeichnet, dass** die Wasseraustrittsstelle eine solche eines Whirlpools ist.

30. Vorrichtung nach einem der Ansprüche 27 bis 29, **dadurch gekennzeichnet, dass** die Filtervorrichtung, gegebenenfalls eine von mehreren, möglichst nahe an einer Wasseraustrittsstelle angeordnet ist.

31. Verfahren zur Gewinnung von im wesentlichen keimfreiem Wasser, insbesondere von legionellenfreiem Wasser, **dadurch gekennzeichnet, dass** Wasser, insbesondere Warmwasser, durch mindestens eine Vorrichtung gemäß einem der vorhergehenden Ansprüche filtriert wird.

## Claims

1. Apparatus (1, 11) for provision of essentially germ-free water, in particular of legionella-free water, having a flow-passing filter housing having an unfiltrate side at a water inlet (2, 12) and a filtrate side at a water outlet (3, 13), within which, as filter, a multiplicity of porous hollow fibres (8, 16) is arranged which are provided in the interior of the apparatus with at least one biocide, **characterized in that**, on the filtrate side of the housing, a flow limiter is provided, the passage orifice of which is smaller than the passage orifice of the still unused filter.

2. Apparatus according to Claim 1, **characterized in that** the biocide is an immobilized biocide having slow-release activity.

3. Apparatus according to Claim 1 or Claim 2, **characterized in that** the hollow fibres (8, 16) are provided for influent flow from the outer surface.

4. Apparatus according to one of the preceding claims, **characterized in that** at least one orifice of the hollow fibres is connected to the filtrate side, the orifice preferably being arranged in a preferably shared base plate (9, 17).

5. Apparatus according to one of the preceding claims, **characterized in that** the filter housing has a total holding volume of about 25 to about 1000 ml, in particular about 50 to about 500 ml.

6. Apparatus according to one of the preceding claims, **characterized in that** it is constructed for a water throughput of about 100 to about 4000 l/h, in particular about 500 to about 3000 l/h.

7. Apparatus according to one of the preceding claims, **characterized in that** its water throughput in the unused state is greater, preferably 1.5 to 4 times as great, than a flow-limiting point in the water system.

8. Apparatus according to one of the preceding claims, **characterized in that** the pores of the hollow fibres have a pore width of about 0.01 to about 0.2 µm, in particular about 0.05 to about 0.2 µm.

9. Apparatus according to one of the preceding claims, **characterized in that** the hollow fibres have an internal diameter of about 10 to about 200 µm, in particular about 50 to about 150 µm.

10. Apparatus according to one of the preceding claims, **characterized in that** the hollow fibres of an apparatus have an outer total surface area of about 0.01 to about 3 m², in particular about 0.05 to about 1.5 m².

11. Apparatus according to one of the preceding claims, **characterized in that** the porous hollow fibres consist of plastic, in particular of polyvinylidene fluoride and/or polyether sulphone.

12. Apparatus according to one of the preceding claims, **characterized in that** the apparatus has a prefilter on the unfiltrate side.

13. Apparatus according to one of the preceding claims, **characterized in that** the hollow fibres are flexible.

14. Apparatus according to one of the preceding claims, **characterized in that** the hollow fibres are constructed so as to be self-supporting.

15. Apparatus according to one of the preceding claims, **characterized in that** the hollow fibre walls consist of a plastic into which the biocide is incorporated.

16. Apparatus according to one of the preceding claims, **characterized in that** the hollow fibres extend from the water inlet (2) to the water outlet (3) and are closed on the unfiltrate side.

17. Apparatus according to one of the preceding claims, in particular according to Claim 16, **characterized in that** the hollow fibres (8) are arranged in the filter housing essentially in parallel.

18. Apparatus according to one of the preceding claims, in particular according to Claim 16 or Claim 17, **characterized in that** about 10 to about 100 hollow fibres, in particular about 30 to about 60 hollow fibres, are provided which are arranged in particular in an elongate filter housing.

19. Apparatus according to one of the preceding claims, in particular according to one of Claims 16 to 18, **characterized in that** the hollow fibres have a length of about 10 to about 200 cm, in particular about 30 to about 100 cm.

20. Apparatus according to one of the preceding claims, in particular according to one of Claims 16 to 19, **characterized in that** the filter housing is a spray tubing (1) or is constructed for introduction into a spray tubing.

21. Apparatus according to one of the preceding claims, in particular according to one of Claims 1 to 15, **characterized in that** both orifices of the hollow fibres (16) are connected to the filtrate side.

22. Apparatus according to one of the preceding claims, in particular according to Claim 21, **characterized in that** the hollow fibres (16) are arranged as loops.

23. Apparatus according to one of the preceding claims, in particular according to Claim 21 or Claim 22, **characterized in that** about 250 to about 1000 hollow fibres, in particular about 500 to about 800 hollow fibres, are provided in the filter housing.

24. Apparatus according to one of the preceding claims, in particular according to one of Claims 21 to 23, **characterized in that** the hollow fibres have a length of about 5 to about 50 cm, in particular about 10 to about 25 cm.

25. Apparatus according to one of the preceding claims, in particular according to one of Claims 21 to 24, **characterized in that** the filter housing is constructed as an exchangeable unit having the hollow fibres, in particular as a cartridge (11).

26. Apparatus according to one of the preceding claims, in particular according to one of Claims 21 to 25, **characterized in that** it is suitable for installation into a spray.

27. Apparatus according to one of the preceding claims, **characterized in that** it is constructed for installation in a water system, in particular into a hot water system, preferably between a water heating point and a water exit point.

28. Apparatus according to Claim 27, **characterized in that** the water exit point is a spray.

29. Apparatus according to Claim 27 or Claim 28, **characterized in that** the water exit point is one such of a whirlpool.

30. Apparatus according to one of Claims 27 to 29, **characterized in that** the filter apparatus, if appropriate one of a plurality, is arranged as near as possible to a water exit point.

31. Process for provision of essentially germ-free water, in particular of legionella-free water, **characterized in that** water, in particular hot water, is filtered through at least one apparatus according to one of the preceding claims.

## Revendications

1. Dispositif (1, 11) d'obtention d'eau essentiellement exempte de germes, en particulier d'eau exempte de légionelles, qui présente un boîtier de filtre qui peut être traversé par l'eau, qui présente un côté non filtré du côté de l'entrée d'eau (2, 12) et un côté filtré du côté de la sortie d'eau (3, 13), à l'intérieur duquel plusieurs fibres creuses poreuses (8, 16) sont disposées pour servir de filtre et sont dotées d'au moins un biocide à l'intérieur du dispositif, **caractérisé en ce que** sur le côté filtré du boîtier est prévu un limiteur de débit dont l'ouverture de passage est plus petite que l'ouverture de passage du filtre non encore usagé.

2. Dispositif selon la revendication 1, **caractérisé en ce que** le biocide est un biocide immobilisé avec effet de dépôt.

3. Dispositif selon la revendication 1 ou la revendication 2, **caractérisé en ce que** les fibres creuses (8, 16) sont prévues pour être balayées par leur surface extérieure.

4. Dispositif selon l'une des revendications précédentes, **caractérisé en ce qu'**au moins une ouverture des fibres creuses est reliée au côté filtré, l'ouverture étant de préférence disposée dans une plaque de base (9, 17) de préférence commune.

5. Dispositif selon l'une des revendications précédentes, **caractérisé en ce que** le boîtier de filtre a un volume global de réception d'environ 25 à environ 1 000 ml et en particulier d'environ 50 à environ 500 ml.

6. Dispositif selon l'une des revendications précédentes, **caractérisé en ce qu'**il est configuré pour un débit d'eau d'environ 100 à environ 4 000 l/h et en particulier d'environ 500 à environ 3 000 l/h.

7. Dispositif selon l'une des revendications précédentes, **caractérisé en ce que** son débit d'eau à l'état non usagé est supérieur et de préférence de 1,5 à 4 fois supérieur à celui de l'emplacement qui limite le débit du système de distribution d'eau.

8. Dispositif selon l'une des revendications précédentes, **caractérisé en ce que** les pores des fibres creuses ont une largeur d'environ 0,01 à environ 0,2 µm et en particulier d'environ 0,05 à environ 0,2 µm.

9. Dispositif selon l'une des revendications précédentes, **caractérisé en ce que** les fibres creuses ont un diamètre intérieur d'environ 10 à environ 200 µm et en particulier d'environ 50 à environ 150 µm.

10. Dispositif selon l'une des revendications précédentes, **caractérisé en ce que** les fibres creuses d'un dispositif ont une surface extérieure totale d'environ 0,01 à environ 3 m² et en particulier d'environ 0,05 à environ 1,5 m².

11. Dispositif selon l'une des revendications précédentes, **caractérisé en ce que** les fibres creuses poreuses sont constituées de matière synthétique et en particulier de poly(fluorure de vinylidène) et/ou de polyéthersulfone.

12. Dispositif selon l'une des revendications précédentes, **caractérisé en ce que** le dispositif présente un pré-filtre sur le côté non-filtré.

13. Dispositif selon l'une des revendications précédentes, **caractérisé en ce que** les fibres creuses sont souples.

14. Dispositif selon l'une des revendications précédentes, **caractérisé en ce que** les fibres creuses sont autoportantes.

15. Dispositif selon l'une des revendications précédentes, **caractérisé en ce que** la paroi des fibres creuses est réalisée en une matière synthétique dans laquelle un biocide est incorporé.

16. Dispositif selon l'une des revendications précédentes, **caractérisé en ce que** les fibres creuses s'étendent entre l'entrée d'eau (2) et la sortie d'eau (3) et sont raccordées au côté non-filtré.

17. Dispositif selon l'une des revendications précédentes, et en particulier selon la revendication 16, **caractérisé en ce que** les fibres creuses (8) sont disposées essentiellement en parallèle dans le boîtier du filtre.

18. Dispositif selon l'une des revendications précédentes, et en particulier selon la revendication 16 ou la revendication 17, **caractérisé en ce qu'** environ 10 à environ 100 fibres creuses et en particulier environ 30 à environ 60 fibres creuses sont prévues et sont disposées en particulier dans un boîtier de filtre allongé.

19. Dispositif selon l'une des revendications précédentes, et en particulier selon l'une des revendications 16 à 18, **caractérisé en ce que** les fibres creuses ont une longueur d'environ 10 à environ 200 cm et en particulier d'environ 30 à environ 100 cm.

20. Dispositif selon l'une des revendications précédentes, et en particulier selon l'une des revendications 16 à 19, **caractérisé en ce que** le boîtier de filtre est un tuyau flexible (1) pour douche ou est configuré pour être incorporé dans un tuyau flexible de douche.

21. Dispositif selon l'une des revendications précédentes et en particulier selon l'une des revendications 1 à 15, **caractérisé en ce que** les deux ouvertures des fibres creuses (16) sont reliées au côté filtré.

22. Dispositif selon l'une des revendications précédentes et en particulier selon la revendication 21, **caractérisé en ce que** les fibres creuses (16) sont disposées sous la forme de boucles.

23. Dispositif selon l'une des revendications précédentes et en particulier selon la revendication 21 ou la revendication 22, **caractérisé en ce qu'**environ 250 à environ 1 000 fibres creuses et en particulier entre environ 500 et environ 800 fibres creuses sont prévues dans le boîtier de filtre.

24. Dispositif selon l'une des revendications précédentes et en particulier selon l'une des revendications 21 à 23, **caractérisé en ce que** les fibres creuses ont une longueur d'environ 5 à environ 50 cm et en particulier d'environ 10 à environ 25 cm.

25. Dispositif selon l'une des revendications précédentes et en particulier selon l'une des revendications 21 à 24, **caractérisé en ce que** le boîtier de filtre est configuré comme entité qui peut être remplacée avec les fibres creuses et en particulier comme cartouche (11).

26. Dispositif selon l'une des revendications précédentes et en particulier selon l'une des revendications 21 à 25, **caractérisé en ce qu'**il est prévu pour être incorporé dans une douche.

27. Dispositif selon l'une des revendications précédentes, **caractérisé en ce qu'**il est configuré pour être incorporé dans un système de distribution d'eau et en particulier un système de distribution d'eau chaude, de préférence entre un emplacement de chauffage de l'eau et un emplacement de sortie de l'eau.

28. Dispositif selon la revendication 27, **caractérisé en ce que** l'emplacement de sortie d'eau est une douche.

29. Dispositif selon la revendication 27 ou la revendication 28, **caractérisé en ce qu'**un emplacement de sortie d'eau est celui d'une baignoire agitée.

30. Dispositif selon l'une des revendications 27 à 29, **caractérisé en ce que** le dispositif de filtration et éventuellement plusieurs dispositifs de filtration sont disposés à proximité aussi étroite que possible d'un emplacement de sortie d'eau.

31. Procédé d'obtention d'eau essentiellement exempte de germes et en particulier d'eau exempte de légionelles, **caractérisé en ce que** l'eau et en particulier l'eau chaude sont filtrées par au moins un dispositif selon l'une des revendications précédentes.
